# DEMANDE DE BREVET EUROPEEN

(11) **EP 1 175 889 A1**
(43) Date de publication de la demande: **30.01.2002**
(21) Numéro de dépôt: 01401973.1
(22) Date de dépôt: 23.07.2001
(51) Int. Cl.: A61K 7/06, A61K 7/155

(54) **Utilisation d'antagonistes des récepteurs des prostaglandines EP-2 et/ou EP-4 pour atténuer ou stopper la pousse des scheveux et des poils dans des préparations cosmétiques**

(30) Priorité: 28.07.2000 FR 0009986
(71) Demandeur: L'OREAL, 75008 Paris (FR)
(72) Inventeur: Bernard, Bruno, 92200 Neuilly-sur-Seine (FR); Michelet, Jean-Françoise, 94000 Creteil (FR); Mahe, Yann, 91390 Morsang-sur-Orge (FR)
(74) Mandataire: Casalonga, Axel

(57) **Abrégé**

La présente invention est relative à une utilisation d'antagonistes des récepteurs des prostaglandines EP-2 et/ou EP-4 pour atténuer, diminuer ou stopper la pousse des cheveux et des poils dans des préparations cosmétiques et au procédé de traitement cosmétique mettant en oeuvre ces compositions.

## Description

La présente invention est relative à une utilisation d'antagonistes des récepteurs des prostaglandines EP-2 et/ou EP-4 pour atténuer, diminuer ou stopper la pousse des cheveux et des poils dans des préparations cosmétiques et au procédé de traitement cosmétique mettant en oeuvre ces compositions.

Il apparaît important dans de nombreuses utilisations telles que l'épilation, pour lutter contre l'hirsutisme et les poils indésirables ou pour empêcher la pousse des cheveux sur un crâne chauve de trouver des composés permettant de préparer des compositions destinées à atténuer, diminuer ou arrêter la pousse des matières kératiniques.

Le brevet WO 96/09806 de Handelman décrit des compositions permettant de réduire la croissance des cheveux contenant une quantité suffisante d'inhibiteur de la protéine kinase C tels que le verapamil, la thioridazine, le curcumin...

Par ailleurs, le document "VII. International Union of Pharmacology Classification of Prostanoid Receptors: Properties, Distribution, and Structure of the Receptors and Their Subtypes" de Coleman et al., Pharmacological Reviews, 1994, Vol 46, mentionne l'existence d'antagonistes des récepteurs aux prostaglandines EP-2 et/ou EP-4.

Le document "Growth regulation of primary human keratinocytes by prostaglandin E receptor EP-2 and EP-3 subtypes" de Konger et al., (Biochimica Biophysica Acta 1401,1998, 221-224) révèle que les récepteurs aux prostaglandines EP-2 et/ou EP-4 sont impliqués dans la régulation de la croissance des cellules épithéliales.

Néanmoins, il est bien connu que le programme de différenciation des kératinocytes de l'épiderme et du follicule pileux sont clairement différents. Ainsi, il est connu que les marqueurs de différenciation telle que les kératines K1 et K10 ne sont pas exprimés dans le follicule pileux et en particulier dans la gaine externe (Lenoir et al., 1988, Dev. Biol. 130: 610-620); que la trichohyaline est exprimée dans le follicule pileux en particulier dans la gaine interne mais pas dans l'épiderme (O'Guin et al., 1992, J. Invest. Dermatol. 98: 24-32); et que la cyclooxygénase de type 1 n'est pas exprimée dans les kératinocytes du follicule pileux mais dans l'épiderme (Michelet. et al., 1997, J. Invest. Dermatol. 108: 205-209).

De plus, il est connu que les kératinocytes de l'épiderme et du follicule pileux se comportent de façon différente en réponse à un même agent pharmacologique. Ainsi, il est connu qu'in vivo le traitement de l'épiderme par l'acide rétinoïque induit une hyperplasie et une spongiose (Griffiths et al., 1993, J. Invest. Dermatol. 101: 325-328) tandis que le traitement du cuir chevelu induit une chute des cheveux (Berth-Jones et al., 1990, Br. J. Dermatol. 122:751-755), et qu'in vitro l'acide rétinoïque, selon la dose utilisée, favorise ou réduit la différenciation de l'épiderme (Asselineau et al., 1989, Dev. Biol. 133:32-335), tandis qu'il provoque l'arrêt de la croissance du follicule pileux (Billoni et al., 1997, Acta Dermatol.Venerol. 77: 350-355). Il est également connu que l'EGF induit une hyperplasie de l'épiderme et simultanément une régression du follicule pileux (Philip et al., 1985, J. Invest. Dermatol. 84: 172-175).

La demanderesse a découvert de manière tout à fait surprenante qu'il était possible de limiter la pousse des poils ou des cheveux par l'utilisation d'antagonistes des récepteurs des prostaglandines EP-2 et/ou EP-4.

La demanderesse a ainsi constaté que l'utilisation conforme à l'invention permettait d'obtenir un effet rapide, et d'utiliser un antagoniste des récepteurs des prostaglandines EP-2 et/ou EP-4 à une concentration faible avec/ou une fréquence d'application faible. Ces agonistes sont capables d'atténuer, de diminuer ou de stopper la pousse des cheveux et des poils et/ou d'augmenter la chute des cheveux et des poils.

Ces composés sont particulièrement peu toxiques et présentent une bonne conservation.

L'utilisation de ces composés permet d'obtenir, notamment par rapport à celles antérieurement connues, des compositions plus efficaces pouvant être utilisées de façon particulièrement aisée, et permettant en outre une élimination facile des compositions par simple rinçage.

Les compositions conformes à l'invention sont par ailleurs particulièrement appropriées sur le plan cosmétique et dermatologique et ne provoquent pas d'irritation du cuir chevelu, même après un contact prolongé, sans rinçage.

Un objet de la présente invention concerne l'utilisation d'antagonistes des récepteurs des prostaglandines EP-2 et/ou EP-4 pour atténuer, diminuer ou stopper la pousse des cheveux et des poils comme agent cosmétique et dermatologique.

Un autre objet de l'invention est constitué par l'utilisation d'agonistes du récepteur des prostaglandines EP-3 pour la préparation d'une composition cosmétique ou dermatologique.

Un autre objet de l'invention est constitué par le procédé de traitement cosmétique ou dermatologique des cheveux et des poils mettant en oeuvre une telle composition.

D'autres objets de l'invention apparaîtront à la lecture de la description et des exemples qui suivent.

L'utilisation d'antagonistes des récepteurs des prostaglandines EP-2 et/ou EP-4 conforme à l'invention permet d'atténuer, de diminuer ou de stopper la pousse des cheveux et des poils. Ces antagonistes des récepteurs des prostaglandines EP-2 et/ou EP-4 vont agir en tant qu'agent cosmétique ou dermatologique permettant d'atténuer, de diminuer ou de stopper la pousse des cheveux et des poils. Ces agonistes sont également capables d'atténuer, de diminuer ou de stopper la pousse des cheveux et des poils.

Les prostaglandines sont des effecteurs biologiques issus d'acide gras polyinsaturés comme par exemple l'acide arachidonique pour PGA₂, PGE₂, PGF_{2α}, TXA₂, ou encore l'acide dihomo γ linolénique pour PGE₁. Les prostaglandines sont impliquées dans de nombreux phénomènes de régulations physiologiques.

Les récepteurs des prostaglandines EP-2 et EP-4 sont des récepteurs de la famille des récepteurs EP qui comprend un très grand nombre de récépteurs différents lorsque on considère les grandes variations dans l'activité tissulaire des prostaglandines.

Un antagoniste est un composé qui va se fixer sur un recepteur et qui ne va pas induire une réponse biologique, contrairement à l'agoniste qui va induire une réponse biologique similaire à celle que l'on obtient avec le ligand naturel qui active cette réponse. Il peut par ailleurs empêcher ou diminuer l'efficacité du ligand naturel qui active normalement cette réponse.

On entend également par poils, les cheveux, les cils, les sourcils et tous les poils d'une manière générale, quelle que soit leur localisation anatomique.

Les composés préférés sont plus particulièrement choisis parmi le AH6809 et le AH23848B.

Le composé AH6809 est décrit dans l'article "Prostanoid Receptors: Structures, Properties, and Functions" Shuh Narumya et al., Physiological Review, 1999, 1203-1204

Le composé AH23848B est décrit dans l'article "Identification of prostaglandin E receptor "EP-2" cloned from mastocytoma cells as EP4 subtype", FEBS Lett, 364, 339-341, 1995

Un autre objet de la présente invention est l'utilisation d'antagonistes des récepteurs des prostaglandines EP-2 et/ou EP-4, dans ou pour la préparation d'une composition cosmétique ou dermatologique à application topique permettant d'atténuer, de diminuer ou de stopper la pousse des cheveux et des poils.

Cette composition cosmétique peut contenir de 0,001 à 10% et de préférence de 0,1 à 5% d'antagonistes en poids par rapport au poids de la composition.

Conformément à l'invention, les compositions cosmétiques peuvent contenir en outre des agonistes du récepteur aux prostaglandine EP-3, tels que le sulprostone, le composé TEI 3356, le composé M&B-28767, la prostaglandine PGE1 décrits dans l'article "Prostanoid Receptors: Structures, Properties, and Functions" Shuh Narumya et al., Physiological Review, 1999, 1203-1204, afin d'augmenter l'effet de chute des cheveux et des poils.

Le milieu cosmétiquement ou dermatologiquement acceptable des compositions selon l'invention, est plus particulièrement constitué d'eau et éventuellement de solvant organique cosmétiquement acceptable.

Les solvants organiques peuvent représenter de 5 à 98% du poids total de la composition. Ils peuvent être choisis dans le groupe constitué par les solvants organiques hydrophiles, les solvants organiques lipophiles, les solvants amphiphiles ou leurs mélanges.

Ainsi la composition peut contenir en outre un milieu cosmétiquement acceptable constitué d'eau ou d'eau et d'au moins un solvant organique choisi dans le groupe constitué par les solvants organiques hydrophiles, les solvants organiques lipophiles, les solvants amphiphiles ou leurs mélanges.

Parmi les solvants organiques hydrophiles, on peut citer par exemple des mono-alcools inférieurs, linéaires ou ramifiés, ayant de 1 à 8 atomes de carbone comme l'éthanol, le propanol, le butanol, l'isopropanol, l'isobutanol; des polyéthylèneglycols ayant de 6 à 80 oxydes d'éthylène; des polyols tels que le propylèneglycol, l'isoprène glycol, le butylèneglycol, le glycérol, le sorbitol; les mono- ou dialkyles d'isosorbide dont les groupements alkyle ont de 1 à 5 atomes de carbone comme le diméthyl isosorbide; les éthers de glycol comme le diéthylène glycol mono-méthyle ou mono-éthyléther et les éthers de propylène glycol comme le dipropylène glycol méthyléther.

Comme solvants organiques amphiphiles, on peut citer des polyols tels que des dérivés de propylèneglycol (PPG), tels que les esters de polypropylèneglycol et d'acides gras, de PPG et d'alcools gras comme le PPG-23 oleyléther et le PPG-36 oléate.

Comme solvants organiques lipophiles, on peut citer par exemple les esters gras tels que l'adipate de diisopropyle, l'adipate de dioctyle, les benzoates d'alkyle.

Les solvants organiques préférés sont choisis dans le groupe constitué par les alcools mono- ou polyfonctionnels, les polyéthylène glycols éventuellement oxyéthylénés, les esters de polypropylène glycol, le sorbitol et ses dérivés, les dialkyls d'isosorbide, les éthers de glycol et des éthers de polypropylène glycol, les esters gras.

Afin que les compositions cosmétiques ou dermatologiques de l'invention soient plus agréables à utiliser (plus douces à l'application, plus nourrissantes, plus émolliantes), il est possible d'ajouter une phase grasse dans le milieu de ces compositions.

La phase grasse représente, de préférence, de 0 à 50% en poids total de la composition.

Cette phase grasse peut comporter une ou plusieurs huiles choisies de préférence dans le groupe constitué par :
- les silicones volatiles ou non-volatiles, linéaires, ramifiées ou cycliques, organo-modifiées ou non, hydrosolubles ou liposolubles,
- les huiles minérales telles que l'huile de paraffine et de vaseline,
- les huiles d'origine animale telles que le perhydrosqualène,
- les huiles d'origine végétale telles que l'huile d'amandes douces, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que l'huile de Purcellin, les isoparaffines,
- les huiles fluorées et perfluorées,
- les esters d'acides gras tels que l'huile de Purcellin.

Elle peut aussi comporter comme matières grasses un ou plusieurs alcools gras, acides gras (acide ferrique) ou cires (paraffine, cire de polyéthylène, Carnauba, cire d'abeilles).

La composition peut également contenir en outre au moins un additif choisi dans le groupe constitué par les gélifiants et/ou épaississants classiques hydrophiles ou lipophiles; des actifs hydrophiles ou lipophiles; des conservateurs; des antioxydants; des parfums; des émulsionnants; des agents hydratants; des agents pigmentants; des dépigmentants; des agents kératolytiques; des vitamines; des émollients; des séquestrants; des tensio-actifs; des polymères; des agents alcanisants ou acidifiants; des charges; des agents anti-radicaux libres; des céramides; des filtres solaires; des répulsifs pour insectes; des agents amincissants; des matières colorantes; des bactéricides; des anti-pelliculaires.

Les quantités de ces différents adjuvants sont celles classiquement utilisées dans les domaines considérés.

Bien entendu, l'homme de l'art veillera à choisir le ou les éventuels composés à ajouter à la composition selon l'invention, de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous toutes les formes galléniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide ou d'une dispersion d'huile dans une phase aqueuse à l'aide de sphérules, ces sphérules pouvant être des nanoparticules polymériques telles que les nanosphères et les nanocapsules, ou mieux, des vésicules lipidiques de type ionique et/ou non-ionique.

Les compositions peuvent avoir l'aspect d'une crème blanche ou colorée, d'une pommade, d'un lait, d'une lotion, d'un sérum, d'une pâte, d'une mousse ou d'un solide.

Elles peuvent éventuellement être appliquées sur la peau sous forme d'aérosol.

Elles peuvent également se présenter sous forme solide, et par exemple sous forme de stick. Elles peuvent être utilisées comme produit de soin et/ou comme produit de maquillage.

La composition peut présenter un pH compris entre 3 et 8.

Un autre objet de la présente invention consiste en un procédé de traitement cosmétique ou dermatologique destiné à atténuer, diminuer ou stopper la pousse des cheveux et des poils, qui consiste à appliquer sur les cheveux et/ou les poils une quantité cosmétiquement ou dermatologiquement efficace d'antagonistes des récepteurs des prostaglandines EP-2 et/ou EP-4.

Selon un autre objet de l'invention le procédé de traitement cosmétique ou dermatologique destiné à atténuer, diminuer ou stopper la pousse des cheveux et des poils consiste également à appliquer sur les cheveux et/ou les poils une composition cosmétique ou dermatologique telle que définie.

Les exemples qui suivent sont destinés à illustrer l'invention sans pour autant présenter un caractère limitatif:

### EXEMPLES DE LOTION ANTIPOUSSE

### Exemple I:

| | |
|---|---|
| AH6809 | 0,03 g |
| Propylène glycol | 20 g |
| Ethanol 95° | 30 g |
| Eau qsp | 100 g |

Cette lotion est appliquée quotidiennement à raison de 10 ml sur le cuir chevelu et ce pendant 2 à 3 mois. On constate alors un net ralentissement de la pousse quotidienne des cheveux et des poils

### Exemple II:

| | |
|---|---|
| AH23848B | 0,15 g |
| Polyglyceryl 3-hydroxylaurylether | 26 g M.A. |
| Hydroxy propyl cellulose vendue sous la | |
| dénomination de Klucell G par la sté HERCULES | 2 g |
| Conservateur | qs |
| Ethanol 95° | 50 g |
| Eau qsp | 100 g |

Cette lotion est utilisée quotidiennement comme un shampooing à raison de 15 g par chevelure avec un temps de pose de l'ordre d'une minute et ceci pendant une période de 4 mois. On observe alors un ralentissement notable de la pousse quotidienne des cheveux

### Exemple III:

| | |
|---|---|
| AH6809 | 0,3 g |
| Propylène glycol | 20 g |
| Ethanol 95° | 30 g |
| Eau qsp | 100 g |

Cette lotion est appliquée quotidiennement à raison de 10 ml sur le cuir chevelu et ce pendant 2 à 3 mois. On constate alors un net ralentissement de la pousse quotidienne des cheveux et des poils

### Exemple IV:

| | |
|---|---|
| AH23848B | 0,15 g |
| Polyglyceryl 3-hydroxylaurylether | 26 g M.A. |
| Hydroxy propyl cellulose vendue sous la | |
| dénomination de Klucell G par la sté HERCULES | 2 g |
| Conservateur | qs |
| Sulprostone | 1 g |
| Ethanol 95° | 50 g |
| Eau qsp | 100 g |

Cette lotion est utilisée quotidiennement comme un shampooing à raison de 15 g par chevelure avec un temps de pose de l'ordre d'une minute et ceci pendant une période de 4 mois. On observe alors un ralentissement notable de la pousse quotidienne des cheveux

### EXPÉRIMENTATION:

Afin d'étudier le comportement du follicule pileux en présence d'antagonistes des récepteurs des prostaglandines EP-2 et/ou EP-4, la demanderesse s'est servie de la méthode dite du "cheveu en survie" du brevet FR 9508465 de L'ORÉAL.

À partir d'une biopsie de scalp, une bande de cuir chevelu assez fine a été isolée à l'aide d'un scalpel. Avec une micropince, le tissu adipeux autour des follicules a été éliminé, en évitant d'endommager le bulbe pilaire. Sous un microscope, le follicule a été coupé avec un scalpel pour le séparer de son environnement épidermique et dermique.

Un des fragments obtenus a été maintenu en culture dans du milieu Williams E, à 37°C, en atmosphère humide en présence de 5% de CO₂ et servira de témoin.

Les autres fragments sont mis dans le même milieu de culture en présence d'antagonistes des récepteurs des prostaglandines EP-2 et/ou EP-4: le AH6809 et le AH23848B

Les fragments en présence des agonistes ainsi maintenus en histoculture réduisent leur taille d'une manière significativement plus importante en comparaison avec le fragment témoin sans antagonistes.

## Revendications

1. Utilisation d'antagonistes des récepteurs des prostaglandines EP-2 et/ou EP-4 comme agent cosmétique permettant d'atténuer, de diminuer ou de stopper la pousse des cheveux et des poils.

2. Utilisation selon la revendication 1, **caractérisée par le fait que** les antagonistes sont choisis parmi le AH6809 et le AH23848B.

3. Utilisation d'antagonistes des récepteurs des prostaglandines EP-2 et/ou EP-4 selon la revendication 1 ou 2, dans ou pour la préparation d'une composition cosmétique à application topique permettant d'atténuer, de diminuer ou de stopper la pousse des cheveux et des poils.

4. Utilisation selon la revendication 3, **caractérisée par le fait que** ladite composition cosmétique contient de 0,001 à 10% et de préférence de 0,1 à 5% d'antagonistes en poids par rapport au poids de la composition.

5. Utilisation selon la revendication 3 ou 4, **caractérisée par le fait que** les compositions cosmétiques peuvent contenir en outre des agonistes du récepteur des prostaglandines EP-3, afin d'accroître la chute des cheveux et des poils.

6. Utilisation selon l'une quelconque des revendications 3 à 5, **caractérisée par le fait que** la composition contient en outre un milieu cosmétiquement acceptable constitué d'eau ou d'eau et d'au moins un solvant organique choisi dans le groupe constitué par les solvants organiques hydrophiles, les solvants organiques lipophiles, les solvants amphiphiles ou leurs mélanges.

7. Utilisation selon la revendication 6, **caractérisée par le fait que** les solvants organiques sont choisis dans le groupe constitué par les alcools mono- ou polyfonctionnels, les polyéthylène glycols éventuellement oxyéthylénés, les esters de polypropylène glycol, le sorbitol et ses dérivés, les dialkyls d'isosorbide, les éthers de glycol et des éthers de polypropylène glycol, les esters gras.

8. Utilisation selon la revendication 6 ou 7, **caractérisée par le fait que** le ou les solvants organiques représentent de 5 à 98% du poids total de la composition.

9. Utilisation selon l'une quelconque des revendications 3 à 8, **caractérisée en ce qu'**elle comprend en outre au moins une phase grasse.

10. Utilisation selon la revendication 9, **caractérisée par le fait que** la phase grasse représente de 0 à 50% du poids total de la composition.

11. Utilisation selon l'une quelconque des revendications 3 à 10,**caractérisée par le fait qu'**elle contient au moins un additif choisi dans le groupe constitué par les gélifiants et/ou épaississants classiques hydrophiles ou lipophiles; des actifs hydrophiles ou lipophiles; des conservateurs; des antioxydants; des parfums; des émulsionnants; des agents hydratants; des agents pigmentants; des dépigmentants; des agents kératolytiques; des vitamines; des émollients; des séquestrants; des tensio-actifs; des polymères; des agents alcanisants ou acidifiants; des charges; des agents anti-radicaux libres; des céramides; des filtres solaires; des répulsifs pour insectes; des agents amincissants; des matières colorantes; des bactéricides; des anti-pelliculaires.

12. Utilisation selon l'une quelconque des revendications 3 à 11, **caractérisée par le fait que** la composition se présente sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide ou d'une dispersion d'huile dans une phase aqueuse à l'aide de sphérules.

13. Utilisation selon l'une quelconque des revendications 3 à 12, **caractérisée par le fait que** la composition a l'aspect d'une crème blanche ou colorée, d'une pommade, d'un lait, d'une lotion, d'un sérum, d'une pâte, d'une mousse ou d'un solide.

14. Utilisation selon l'une quelconque des revendications 3 à 13, **caractérisée par le fait que** la composition présente un pH compris entre 3 et 8.

15. Procédé de traitement cosmétique destiné à attenuer, diminuer ou stopper la pousse des cheveux et des poils, **caractérisé par le fait qu'**il consiste à appliquer sur les cheveux et les poils une quantité cosmétiquement efficace d'antagonistes tels que défini dans l'une quelconque des revendications 1 à 2.

16. Procédé de traitement cosmétique destiné à attenuer, diminuer ou stopper la pousse des cheveux et des poils, **caractérisé par le fait qu'**il consiste à appliquer sur les cheveux et les poils une composition cosmétique telle que définie dans l'une quelconque des revendications 3 à 14.
